# EUROPEAN PATENT APPLICATION

(11) **EP 4 574 078 A1**
(43) Date of publication of application: **25.06.2025**
(21) Application number: 24220985.6
(22) Date of filing: 18.12.2024
(51) Int. Cl.: A61B 34/20, A61B 34/30, A61B 90/00, A61B 34/10

(54) **SYSTEM FOR SETTING THE ORIENTATION OF A 3D MODEL OF AN ANATOMICAL PART**

(30) Priority: 20.12.2023 IT 202300027459
(71) Applicant: AMDengineering S.r.l., 25124 Brescia (BS) (IT); I2ING S.r.l., 25016 Ghedi (BS) (IT)
(72) Inventor: Buccelli, Andrea, 25010 San Zeno Naviglio (IT); Faustini, Giuliano, 25030 Erbusco (IT); Tosoni, Fabio, 25050 Passirano (IT); Stefanelli, Fabio, 25016 Ghedi (IT)
(74) Representative: Perani & Partners S.p.A.

(57) **Abstract**

A system (1) for setting the orientation of a 3d model (2) of an anatomical part comprises a 3d model (2) of an anatomical part having a model reference frame (MF); a tracker device (3) to be attached to a surgical instrument (100) active on a surgical object (101) having a surgery reference frame (SF), the tracker device (3) comprises sensing means (4) to detect the orientation of the tracker device (3) in a fixed reference frame (FF) to define a tracker device reference frame (TF), the system (1) further comprises a processing unit (10) comprising a video output module (11) to be placed in signal communication with viewing means (200) to display the 3d model (2) in a viewing reference frame (VF); a memory module (12) configured to hold the 3d model (2); a signal acquisition module (13) to receive the orientation signal (OS) from the tracker device (3); a viewpoint shift module (14) configured to adjust the viewing reference frame (VF) as a function of the orientation signal (OS) so that the orientation of the viewing reference frame (VF) with respect to the model reference frame (MF) is the same as the orientation of the tracker device reference frame (TF) with respect to the surgical reference frame (SF).

## Description

### BACKGROUND

Embodiments of the present disclosure relate to a system for setting the orientation of a 3d model of an anatomical part. In the context of the present disclosure, "anatomical part" should be intended as any arbitrary portion of either a human or animal body, comprising one or more internal organ, anatomical structure or portions thereof. Such system is generally useful in the field of surgery, either in the preoperative phase to plan a surgery or during the actual procedure as a visual aid to the surgeon. The system can also be employed as a training tool for physicians.

Generating a 3d model of an anatomical part is generally known in the state of the art. Given the recent advances in 3d processing computing power, these 3d models have become more and more detailed, to the point that they can be used as surgery aids, for example during a laparoscopic surgery where the ability of the surgeon to visually inspect the actual area being operated is limited. Having access to an accurate 3d model allows the surgeon to know if any structure is placed behind an area where an incision is to be performed, for example an artery, so that the surgeon is able to correctly plan and execute the incision without touching the artery behind, thus increasing the safety and decreasing the invasiveness of the surgical procedure.

Disadvantageously, the 3d model usually is not spatially oriented in the same way as the actual point of view of the surgeon which, in case of a laparoscopy, is defined by the tip of the laparoscope. Therefore, in order to align the 3d model with the viewpoint of the surgeon, manual intervention is required. This limits the usefulness of the 3d model as a surgical aid.

### BRIEF DESCRIPTION OF THE INVENTION

The object of the present disclosure is to provide a system for setting the orientation of a 3d model of an anatomical part that is more reliable when used as a visual aid during surgery.

A further object of the present disclosure is to provide a system for setting the orientation of a 3d model of an anatomical part that can used as a training tool for surgeons.

These and other objects are fulfilled by a system for setting the orientation of a 3d model of an anatomical part as defined in any of the accompanying claims.

A first aspect of the disclosure is therefore directed to a system for setting the orientation of a 3d model of an anatomical part. The system comprises a 3d model of an anatomical part having a model reference frame.

The system also comprises a tracker device configured to be attached to a surgical instrument active on a surgical object having a surgery reference frame. The tracker device comprises sensing means, which are configured to detect the orientation of the tracker device in a fixed reference frame to define a tracker device reference frame. Data transmission means are configured to emit an orientation signal representative of the orientation of the tracker device reference frame.

The system also comprises a processing unit, which has a video output module configured to be placed in signal communication with viewing means to display the 3d model in a viewing reference frame.

The processing unit also comprises a memory module configured to hold the 3d model.

A signal acquisition module is configured to be placed in signal communication with the tracker device to receive the orientation signal.

A viewpoint shift module is configured to adjust the viewing reference frame as a function of the orientation signal so that the orientation of the viewing reference frame with respect to the model reference frame is the same as the orientation of the tracker device reference frame with respect to the surgical reference frame.

The system of the present disclosure achieves the above mentioned objectives. Indeed, the use of the viewpoint shift module, together with the input from the tracker device, allows to render an image of the 3d model that has substantially the same point of view than the image captured by the actual surgical instrument, allowing the surgeon to use the 3d model as a "map" to perform the surgical procedure.

Furthermore, matching the movement of a surgical instrument with a rendered image allows the system to be used in training surgeons, either as general training or for a specific procedure.

### BRIEF DESCRIPTION OF THE DRAWINGS

Further details and specific embodiments will refer to the attached drawings, in which:
- Figure 1 is a schematic view of a system for setting the orientation of a 3d model of an anatomical part according to an embodiment of the present disclosure;
- Figure 2 is a schematic perspective view of a component of the system of Figure 1; and

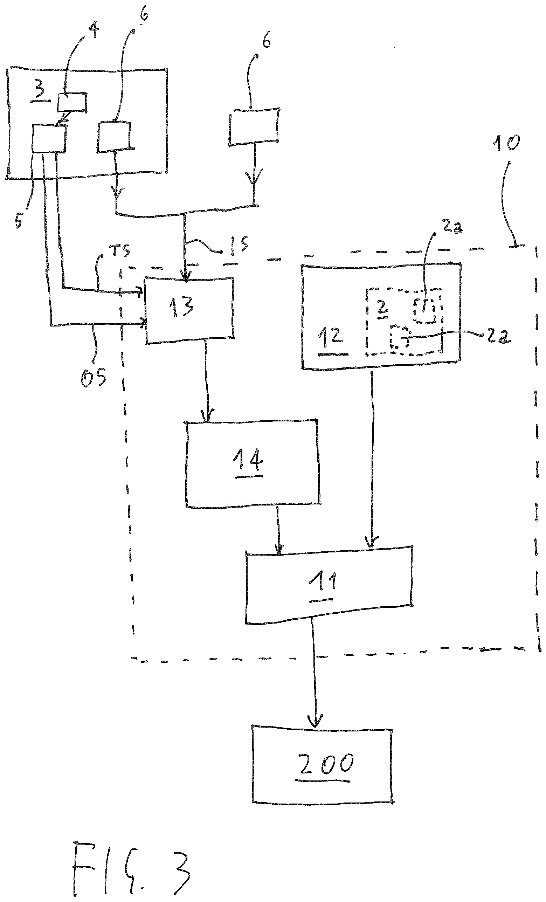

- Figure 3 is a block diagram illustrating the functioning of the system of Figure 1.

### DETAILED DESCRIPTION

The following description of exemplary embodiments refer to the accompanying drawings. The same reference numbers in different drawings identify the same or similar elements. The following detailed description does not limit the invention. Instead, the scope of the invention is defined by the appended claims.

Reference throughout the specification to "one embodiment" or "an embodiment" means that a particular feature, structure, or characteristic described in connection with an embodiment is included in at least one embodiment of the subject matter disclosed. Thus, the appearance of the phrases "in one embodiment" or "in an embodiment" in various places throughout the specification is not necessarily referring to the same embodiment. Further, the particular features, structures or characteristics may be combined in any suitable manner in one or more embodiments.

Referring to the attached figures, it will now be described a system 1 for setting the orientation of a 3d model 2 of an anatomical part. The system is intended to be used with a surgical instrument 100, for example a laparoscope or, more preferably, a robotic arm configured to hold a surgical tool such as a laparoscope. The surgical instrument 100 will not be described in detail, since it is not itself part of the present invention, and may be of a type known to the person skilled in the art.

In use, the surgical instrument 100 is active on a surgical object 101, which has a surgery reference frame "SF". The surgical object 101 may be a physical artificial replica of an anatomical part or a computer simulation thereof, in which case it may form part of the present invention. Alternatively, the surgical object 101 may be an actual anatomical portion either from a non-living person, for example used for training purposes, or from a live patient. The surgery reference frame "SF" is defined with respect to a fixed reference frame, for example the surgery room, and reflects the orientation of the surgical object 101 with respect to the fixed reference frame.

The system 1 also comprises a 3d model 2 of an anatomical part. Such 3d model 2 has a model reference frame MF defined therein. Preferably the 3d model 2 is prepared in advance from the results of medical exams conducted by the use of medical imaging devices, for example computed axial tomography scan (CAT scan) machines. The 3d model 2 can be prepared manually, so as to respond to the requirements of the surgeon, for an operation on a live patient. Alternatively, the 3d model 2 can be prepared automatically by a neural network, and can be used as a training tool for physicians.

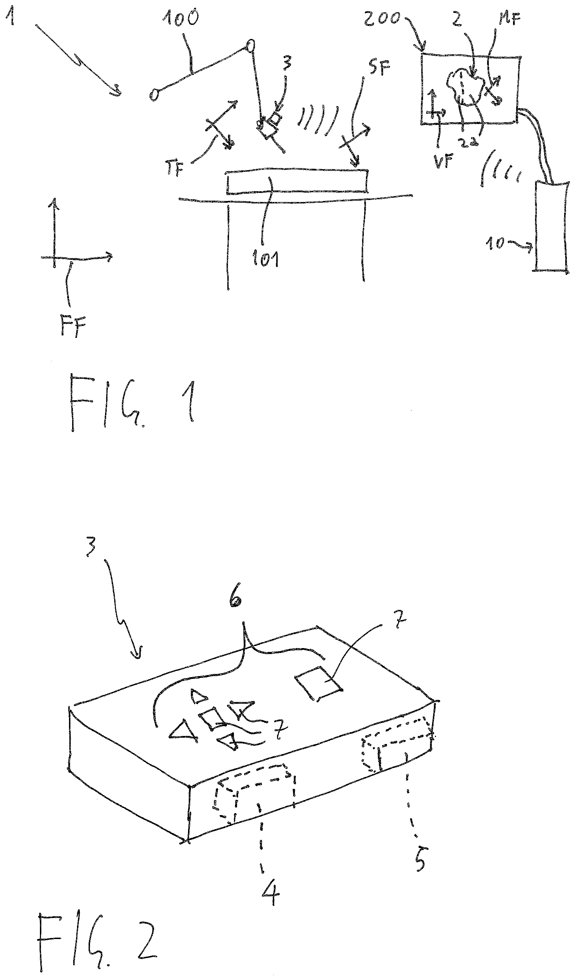

The system 1 further comprises a tracker device 3, schematically shown in figure 2, which is designed to be attached to the surgical instrument 100 described above. Specifically, the tracker device 3 has the function of tracking the orientation and the position of the surgical instrument 100. To that purpose, the tracker device 3 comprises sensing means 4 configured to detect the orientation of the tracker device 3 in a fixed reference frame FF, in order to define a tracker device reference frame TF. The sensing means 4 can comprise an appropriate number of gyroscopes and/or accelerometers, which are themselves known to the person skilled in the art and therefore will not be further described.

The tracker device 3 also comprises data transmission means 5, which are configured to emit an orientation signal OS representative of the orientation of the tracker device reference frame TF. The data transmission means 5 can be either of wired or wireless type, with the wireless type being preferred because of clutter reduction considerations. Additionally, having wireless data transmission means 5 simplifies the use of the system 1 in a sterile setting, as it is not necessary to route a cable from a sterile to a non-sterile area.

The tracker device 3 is also configured to emit a translation signal TS, which is representative of a translation of the tracker device 3, and therefore of the surgical instrument 100, with respect to the fixed reference frame FF. Preferably, the translation is detected by the above cited sensing means 4, and the translation signal TS is routed by the data transmission means 5.

According to the present disclosure, the orientation signal OS is updated substantially in real time. Likewise, the translation signal TS is updated substantially in real time.

Also according to the present disclosure, the system 1 comprises a processing unit 10. In the following part of the present the processing unit 10 will be described as comprising several modules. This is done for ease of description only, and it should not be considered as a structural limitation of the processing unit 10. Indeed, the processing unit 10 can be implemented as hardware, software or the combination of both. The implementation can be either on a local machine, distributed over a network or even a cloud computing solution.

The processing unit 10 comprises a video output module 11, which is configured to be placed in signal communication with viewing means 200 in order to display the 3d model 2. Preferably the viewing means 200 comprise a screen (not shown), that can be either exclusively tasked to the processing unit 10 or, more preferably, part of the general equipment of a surgical room, such as for example a screen associated with the surgical instrument 100. It should be noted that the 3d model 2 is displayed on the viewing means 200 in a viewing reference frame VF, which can be manipulated and adjusted as described in a following part of the present disclosure, so as to change the view angles and the zoom level.

The processing unit 10 further comprises a memory module 12, which is adapted to hold the 3d model 2. Indeed, prior to the surgical procedure (whether real or simulated), the 3d model 2 is saved on the memory module 12, from which it can then be retrieved as needed. For example, the memory module 12 can be implemented through widely available electronic storage, such as for example an hard disk, a solid state drive or a combination of both.

A signal acquisition module 13 is configured to be placed in signal communication with the tracker device 3, to receive the orientation signal OS. Also, the signal acquisition module 13 is adapted to receive the translation signal TS.

The processing unit 10 further comprises a viewpoint shift module 14, which has the function of performing adjustments to the viewing reference frame VF.

According to the present invention, the viewing reference frame VF can be adjusted at least as a function of the orientation signal OS, so that the orientation of the viewing reference frame VF with respect to the model reference frame MF is the same or substantially the same as the orientation of the tracker device reference frame TF with respect to the surgical reference frame SF.

Additionally, the viewpoint shift module 14 is configured to adjust a position of the viewing reference frame VF with respect to the model reference frame MF as a function of the translation signal TS.

Advantageously, these adjustments of the viewing reference frame VF allow to view the 3d model 2 substantially from the same view angle as the surgical instrument 100 views the surgical object 101. In case of a laparoscopy, for example, the surgical instrument 100 is equipped with a camera (not shown) which produces a real image that can be directly compared to the corresponding image of the 3d model 2 and be used as a visual aid during a surgical procedure.

It should be noted that the viewpoint shift module 14 is can also be configured to adjust a zoom factor of the viewing means 200 as a function of either the translation signal TS. In other words, if the surgical instrument moves closer or farther from a viewing area of the surgical object 101, this can be matched as moving the viewpoint closer/farther from the 3d model 2 on the viewing means 200. Alternatively, if the surgical instrument 100 is provided with image acquisition means (i.e. a camera, not shown), the acquired image can be zoomed either optically or digitally, and these changes can be matched on the viewpoint of the viewing means 200.

It should also be noted that the viewpoint shift module 14 is configured to update the viewing reference frame VF substantially in real time. Advantageously, in this way the viewpoint shift module 14 allows to generate an image on the viewing means 200 that perfectly matches the actual image acquired by the surgical instrument 100.

According to the present disclosure, the 3d model 2 may comprise a plurality of layers 2a, each representative of a respective anatomical structure part of the surgical object 101. The processing unit 10 can then be configured to show/hide each layer 2a on the viewing means 200. Preferably, the processing unit 10 is also configured to set a transparency value for each layer 2a. Advantageously, this allows the image of the 3d model 2 to be superimposed onto the actual image acquired by the surgical instrument 100, further improving its usefulness as a visual aid during surgery.

Optionally the system 1, preferably the processing unit 10, is able to perform advanced processing of the medical data part of the 3d model 2. For example, it is possible to display the organs which are represented by the 3d model 2 with a virtual plane, allowing the surgeon to plan and virtually execute the operation on the 3d model 2 prior to the actual surgery. The blood vessels that will need to be closed can be identified, while the total remaining volume of each affected organ can be calculated, as these are important parameters for assessing the viability of the post-surgery organs.

The system 1 also comprises input means 6, which are configured to emit an input signal IS. The viewpoint shift module 14 of the processing unit 10 is then configured to adjust the viewing reference frame VF as a function of the input signal IS

In detail, the input means comprise a 3d pointing device (not shown) which is used to calibrate the tracker device 3 and/or the entire system 1 prior to its use.

Furthermore, the input means 6 may comprise one or more buttons 7 placed on the tracker device 3. Specifically, if the tracker device 3 is designed to be associated to a manual surgical instrument 101, the buttons 7 are present and perform various functions. For example, the buttons 7 can be used to decouple the viewing reference frame VF from the tracker device reference frame TF. This can allow the surgeon to move the surgical instrument 101 without a corresponding movement of the image on the viewing means 200, for example to perform a calibration step before starting a surgical procedure. Alternatively, the buttons 7 can send commands to the processing unit 10 so that the viewing reference frame VF is adjusted without the need to move the surgical instrument 100, so that the surgeon can inspect the area from another angle on the viewing means 200 without needing to perform the corresponding movement.

## Claims

1. System (1) for setting the orientation of a 3d model (2) of an anatomical part, **characterized in that** it comprises:
- a 3d model (2) of an anatomical part having a model reference frame (MF);
- a tracker device (3) configured to be attached to a surgical instrument (100) active on a surgical object (101) having a surgery reference frame (SF), the tracker device (3) comprising:
- sensing means (4) configured to detect the orientation of the tracker device (3) in a fixed reference frame (FF) to define a tracker device reference frame (TF),
- data transmission means (5) configured to emit an orientation signal (OS) representative of the orientation of the tracker device reference frame (TF);
- a processing unit (10) comprising:
- a video output module (11) configured to be placed in signal communication with viewing means (200) to display the 3d model (2) in a viewing reference frame (VF);
- a memory module (12) configured to hold the 3d model (2);
- a signal acquisition module (13) configured to be placed in signal communication with the tracker device (3) to receive the orientation signal (OS);
- a viewpoint shift module (14) configured to adjust the viewing reference frame (VF) as a function of the orientation signal (OS) so that the orientation of the viewing reference frame (VF) with respect to the model reference frame (MF) is the same as the orientation of the tracker device reference frame (TF) with respect to the surgical reference frame (SF).

2. System (1) according to the previous claim, wherein the orientation signal (OS) is updated substantially in real time.

3. System (1) according to any one of the previous claims, wherein the tracker device (3) is configured to also emit a translation signal (TS) representative of a translation of the tracker device (3) with respect to the fixed reference frame (FF), the viewpoint shift module (14) being configured to adjust a position of the viewing reference frame (VF) with respect to the model reference frame (MF) as a function of the translation signal (TS).

4. System (1) according to the previous claim, wherein the viewpoint shift module (14) is configured to adjust a zoom factor of the viewing means (200) as a function of either the translation signal (TS) or either an optical or digital zoom of image acquisition means connected to or part of the surgical instrument (100).

5. System (1) according to any one of the previous claims, wherein the viewpoint shift module (14) is configured to update the viewing reference frame (VF) substantially in real time.

6. System (1) according to any one of the previous claims, wherein the 3d model (2) comprises a plurality of layers (2a) each representative of a respective anatomical structure part of the surgical object, the processing unit (10) being configured to show/hide each layer (2a) on the viewing means (200).

7. System (1) according to any one of the previous claims, wherein the data transmission means (5) is wireless.

8. System (1) according to any one of the previous claims, further comprising input means (6) configured to emit an input signal (IS) representative of the state of the input means (6), the viewpoint shift module (14) being configured to adjust the viewing reference frame (VF) as a function of the input signal (IS).

9. System (1) according to the previous claim, wherein the input means (6) comprises one or more buttons (7) placed on the tracker device (3).

10. System (1) according to claim 8 or 9, wherein the input means (6) comprises a 3d pointing device.
